# EUROPEAN PATENT SPECIFICATION

(11) **EP 3 256 092 B1**
(45) Date of publication and mention of the grant of the patent: **11.10.2023**
(21) Application number: 15882199.1
(22) Date of filing: 10.02.2015
(51) Int. Cl.: A61J 7/00, A61J 7/04

(54) **DRUG DISPENSER**
ARZNEIMITTELSPENDER
DISTRIBUTEUR DE MÉDICAMENT

(43) Date of publication of application: 20.12.2017
(73) Proprietor: Hewlett Packard Enterprise Development LP, Houston, TX 77070 (US)
(72) Inventor: DOSHI, Parag, Marietta, Georgia 30068 (US); KAMALAKANTHA, Chandra, Plano, Texas 75024 (US)
(74) Representative: Gill Jennings & Every LLP
(86) International application number: PCT/US2015/015146
(87) International publication number: WO 2016/130109

(56) References cited:
- WO-A1-02/081015
- US-A- 4 682 299
- US-A1- 2003 222 090
- US-A1- 2007 156 282
- US-A1- 2008 179 387
- US-A1- 2010 228 566
- US-A1- 2013 304 255
- US-B2- 7 502 664

## Description

### BACKGROUND

Drug misuse is a widespread issue that may be further exacerbated globally when considering the developing countries that struggle for a majority literacy rate. In these cases, patients requiring medicine of a precise dose frequently fail to administer the dosage properly. Even in developed countries, patients often forget to take their medicine, or they may intentionally raise dosage assuming quicker healing rates. Such measures lead to waste, an increase in cost, and can result in danger to one's health.

WO 02/081015 (A1) discloses an apparatus for dispensing medication to a patient, said apparatus comprising: (a) an electronic medicament dispenser (2) with one or more data-transfer elements; (b) a docking station (6) with one or more corresponding data-transfer elements; and (c) a wireless communications module (10) for connecting said docking station to a public communications network, whereby data may be transmitted remotely between said medicament dispenser and a remote processing node via said public communications network.

US 2013/304255 (A1) discloses a system and apparatus for displaying drug interaction and side effects on a drug storage container. The system includes a server and a plurality of medicine dispensing devices communicatively connectable to the server. Each of the dispensing devices includes a medicine storage portion having a discharge port, a dispensing assembly configured to selectively receive the pills from the discharge port, an electronic display screen, and a control system. The control system is configured to associate a medicine stored in the medicine storage portion of the medicine dispensing device, generate a list of potential drug interactions associated with the medicine and a second medicine associated with a user, receive, from the server, the list of potential drug interactions associated with the medicine, and display the list of potential drug interactions on the electronic display screen of the first medicine dispensing device.

### BRIEF DESCRIPTION OF THE DRAWINGS

Some examples of the present application are described with respect to the following figures:
FIG. 1 is a block diagram of an example dispenser for administering drugs to a patient; and
FIG. 2 is a block diagram of a computing device for providing drugs to a patient.

### DETAILED DESCRIPTION

As described above, improper administration of drugs to patients is a prevalent problem. For example, a patient may forget to take their medicine at the appropriate time or may take the incorrect amount of their medicine. In some cases, patients may even use a drug beyond its expiration date.

A drug dispenser, as described herein, may be used to administer the correct amount of a drug to the correct patient at the correct time. The dispenser may include a processor and a memory (e.g., an Erasable Programmable Read-Only Memory (EPROM) chip) that may be used to administer the proper dosage to a particular patient at the appropriate time. The dispenser may actively communicate with various medical systems (e.g., doctors' systems, pharmacists' systems, etc.) to communicate any suitable information related to the medicine and/or the patient, such as remotely updating the dispenser with additional or modified instructions relating to the drug, sending information about the administration of the drug (e.g., a time and frequency of actual administration of the drug to the patient) from the dispenser to an associated medical system (e.g., to the related doctor's system for monitoring of the patient), and the like.

In some examples, the dispenser may include one or more processors in communication with a server that is accessible to one or more doctors capable of prescribing drugs to one or more patients as well as one or more pharmacists capable of providing drugs to one or more patients. In some examples, the server may communicate with one or more associated dispensers for a plurality of patients, one or more doctors' systems managing their associated patients' medical information, and/or one or more pharmacists' systems managing their associated patients' medical information. The processor(s) in the dispenser may receive any suitable information associated with the dispenser and/or the patient. For example, instructions relating to administration of the drug to the patient may be received. The instructions may be received when the dispenser is programmed for the appropriate patient and/or drug and may be received either locally or remotely. The instructions may specify any information relating to the conditions under which the drug is to be administered to the patient, such as a timing associated with the administration of the drug to the patient (e.g., drug to be administered every 12 hours), an amount of the drug to be administered for each dosage, an identity of the patient to whom the drug is to be administered, and the like. The dispenser may include a timer in communication with the processor(s). The timer may monitor the timing associated with the administration of the drug based on the instructions and may provide a notification to alert a patient when it is time to take a dose of the drug. The dispenser may also include an identifier mechanism in communication with the processor(s). The identifier mechanism may determine the identity of any person attempting to access the drug in the dispenser to ensure that it is the correct person (e.g., the patient) that is attempting to access the drug. For example, the identifier mechanism may be a biometric scanner, such as a fingerprint scanner, and if the patient is alerted that it is time to take their medication, the timer may provide the alert, the patient may provide their fingerprint using the identifier mechanism, and the dispenser may provide the appropriate amount of the drug if the fingerprint is identified as the patient's fingerprint.

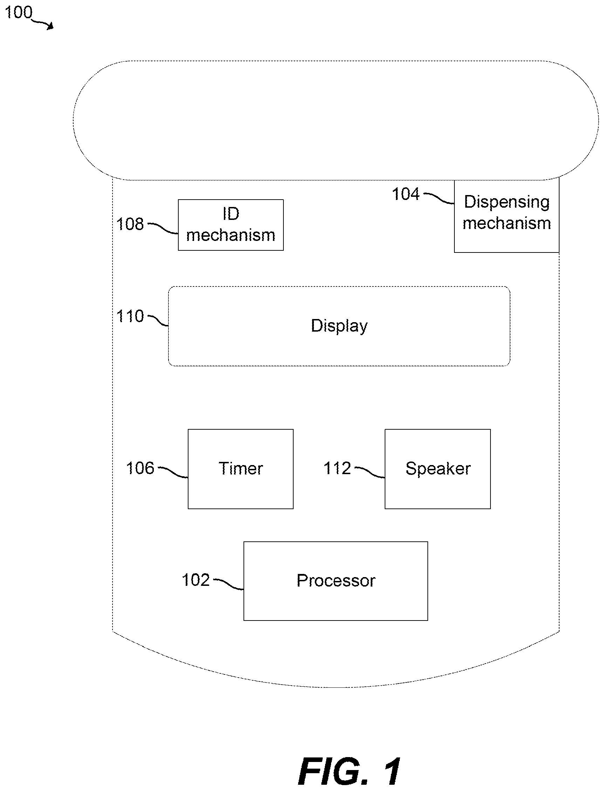

Referring now to the figures, FIG. 1 is a block diagram of an example dispenser 100 for administering drugs to a patient. Dispenser 100 may administer a drug contained within dispenser 100 at the appropriate time, in the appropriate amount, and to the appropriate person based on instructions used to program dispenser 100 such that dispenser 100 operates based on those instructions. Dispenser 100 may be capable of dispensing any type of drug. For example, dispenser 100 may be able to dispense a drug in pill form, liquid form, a drug dispensed through injection, and the like. In some examples, dispenser 100 may include a radio-frequency identification (RFID) chip that may be used to locate dispenser 100 (e.g., if the patient misplaces dispenser 100). Dispenser 100 may be capable of storing and administering any number and/or type of drugs and may be able to prevent cross-drug contamination such that a patient does not mix drugs that may be harmful when mixed. For example, dispenser 100 may administer each drug it stores at the appropriate times according to instructions relating to each drug and any possible harmful side effects associated with mixing the drugs.

Processor 102 may be any suitable processor capable of administering drugs to a patient based on instructions used to program processor 102. The instructions may be any suitable instructions for properly administering a drug to a patient (e.g., timing of when and/or how frequently to administer the drug, who the drug is to be administered to, the appropriate amount of the drug for each dose, etc.). Processor 102 may be in communication with a server that is accessible to one or more doctors who can prescribe drugs to patients and/or one or more pharmacists who can provide drugs to patients based on instructions from one or more doctors. For example, a doctor may use an application associated with the server to prescribe a drug to a patient and to provide instructions for administering the drug to the patient (e.g., instructions for administering proper dosage, timing, patient, etc.). In some examples, the dosage and/or timing may be strictly and/or flexibly set by a doctor, or a doctor may allow or disallow a pharmacist to adjust the prescription (e.g., adjust within certain parameters). In some examples, a doctor may use the application to adjust and/or reprogram dispenser 100 remotely. A pharmacist may also use an application associated with the server to fill the prescription according to the doctor's instructions and may use the instructions to properly program dispenser 100 to operate based on the doctor's instructions and/or reprogram dispenser 100 remotely. In some examples, a pharmacist may use the application to access medical records for a patient to determine whether the patient may be prescribed generic drugs. In some examples, a pharmacist may use the application to access insurance information associated with a patient. A patient may also use an application associated with the server to access any suitable information associated with the patient's prescription and to specify user preferences associated with the patient's prescription (e.g., generic drugs versus brand name drugs, preferred pharmacies, timing for administering drug, etc.). For example, a patient may specify a preference to take a drug before bedtime, and a doctor and/or pharmacist may take this preference into consideration when providing and/or programming dispenser 100 with instructions. The applications used by doctor(s), pharmacist(s), and/or patient(s) may be any suitable application capable of accessing information associated with prescriptions and other medical information (e.g., medical records for patients, insurance information, etc.) available on one or more servers managing the information (e.g., software as a service (SaaS)). In some examples, these applications may run on a cloud server.

Processor 102 may be actively in communication with the server managing information associated with medical entities, such as doctors, pharmacists, patients, insurance companies, and the like. Processor 102 may receive information (e.g., revised instructions for administration of a drug) from and send information associated with dispenser 100 to the server for various reasons. For example, processor 102 may send information about the administration of a drug to a patient, such as a timing related to when the patient received a dosage, and this information may be used for various reasons by various entities. For example, the information may be used to monitor a patient's consumption of the drug (e.g., to ensure the patient is taking the medication properly, for regulatory purposes to ensure that drugs are not being abused, etc.). In some examples, information sent between dispenser 100 and the server may be encrypted such that the information is securely transmitted, and the information may be decrypted when it arrives at its appropriate destination.

In some examples, processor 102 may be programmed with a compensation algorithm that may compensate for missed doses by adjusting the instructions for administering the drug for one or more doses subsequent to a missed dose. For example, if a patient misses a dose, processor 102 may compensate for the missed dose by adjusting the timing for the next dose, adjusting the amount of the drug for the next dose, and the like.

In some examples, processor 102 may have machine-learning functionality and may be able to learn an individual patient's behavior. For example, processor 102 may learn that a particular patient tends to take their medicine 30 minutes late and may provide notifications to the patient about the next dosage 30 minutes ahead of the scheduled dosage.

A wearable device in communication with processor 102 is capable of performing bio-sensor monitoring of a patient and sending information associated with the bio-sensor monitoring from the wearable device to processor 102. Processor 102 may use the information to adjust the administration of the drug accordingly. For example, if bio-sensor monitoring shows information relating to a patient's alcohol consumption, processor 102 may delay the administration of the drug until the patient's blood alcohol level drops to a safe level. A patient's biometrics is monitored to determine whether a patient took their medication and may send out additional notifications to the patient until the patient takes the medication.

In some examples, processor 102 may accommodate for user error. For example, processor 102 may allow a patient to access an additional dosage of medication for an accidental missed dose (e.g., an accidental dropped pill). In some examples, processor 102 may notify a patient that they already took their medication if the patient attempts to access additional medication, or may administer a placebo.

Dispensing mechanism 104 may be any suitable mechanism for dispensing the appropriate amount of a drug to a patient based on the instructions associated with administering the drug. For example, if the instructions indicate that two pills are to be taken for each dose, dispensing mechanism 104 may dispense the two pills at the appropriate time.

Timer 106 may be in communication with processor 102 and may be any suitable timing mechanism capable of monitoring timing of the administration of a drug. For example, timer 106 may be a clock tracking the elapsed time since the last time the drug was administered. In some examples, timer 106 may be integrated with a network time synchronization such that timer 106 may not be tampered with. Timer 106 may notify a patient about various events and/or reminders, such as notifying the patient when it is time to take the drug, notifying the patient about additional instructions relating to the drug (e.g., that the drug is not to be taken on an empty stomach), and the like. The notification may be in any suitable format. For example, timer 106 may provide a notification in the form of an email, a text, a phone call, a vibration on dispenser 100, and the like. In some examples, the notification may be provided using a wearable device (e.g., a smart watch) that is in communication with dispenser 100. In some examples, the notification may be an audible alert provided using speaker 112, which may be any suitable speaker in communication with processor 102 and timer 106. In some examples, the notification may be a visual notification provided on display 110, which may be any suitable display (e.g., a light emitting diode (LED) display) in communication with processor 102 and timer 106. The notification may be useful for drugs having complicated timing for administration. In some examples, timer 106 may monitor the life of the drug in dispenser 100 to prevent the administration of the drug beyond its expiration date.

Identifier mechanism 108 of dispenser 100 may be any suitable device capable of identifying a person attempting to access the drug in dispenser 100. For example, identifier mechanism 108 may be a biometric scanner (e.g., a fingerprint scanner), a touchpad that may be used to input a personal identification number (PIN) or a password allowing access to the drug, and the like. Identifier mechanism 108 may identify a person attempting to access the drug, determine whether the person is the patient to whom the drug was prescribed, and allow access to the drug if the patient is identified (e.g., if a fingerprint received matches a fingerprint of the patient) and if it is time for the patient to take the drug.

Display 110 of dispenser 100 may be any suitable display (e.g., a LED display). Display 110 may be a programmable display that may display any suitable information, such as instructions associated with the prescription, instructions relating to how the dispenser may be returned (e.g., in order for the patient to get their deposit back), personalized information specified by the patient (e.g., the patient's name), and the like.

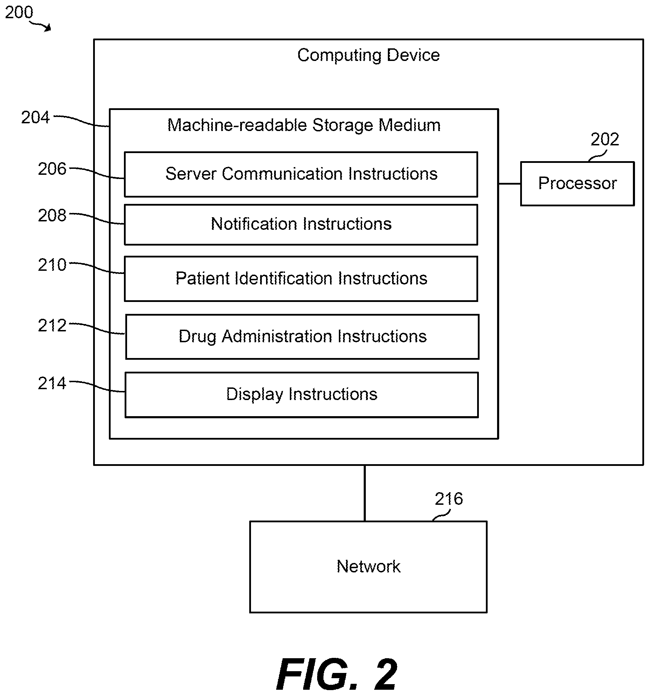

FIG. 2 is a block diagram of an example computing device 200 for providing drugs to a patient. Computing device 200 may be a drug dispenser (e.g., dispenser 100 of FIG. 1). Computing device 200 may receive rules relating to administration of a drug to a patient and may provide the drug based on the rules.

Computing device 200 may be, for example, a web-based server, a local area network server, a cloud-based server, a notebook computer, a desktop computer, an all-in-one system, a tablet computing device, a mobile phone, an electronic book reader, a printing device, or any other electronic device suitable for providing drugs to a patient. Computing device 200 may include a processor 202 and a machine-readable storage medium 204. Computing device 200 may use rules received from a server to administer drugs to the appropriate patient at the correct time and dosage amount.

Computing device 200 may be connected to and in communication with other computing devices, servers, systems, and the like (e.g., computing devices and/or systems of doctors, pharmacists, patients, etc.) either directly or using network 216. Network 216 may be any suitable network. In some examples, one or more portions of network 216 may include an ad hoc network, an intranet, an extranet, a virtual private network (VPN), a local area network (LAN), a wireless LAN (WLAN), a wide area network (WAN), a wireless WAN (WWAN), a metropolitan area network (MAN), a portion of the Internet, a portion of the Public Switched Telephone Network (PSTN), a cellular telephone network, or any other type of network, or a combination of two or more such networks.

Processor 202 is a tangible hardware component that may be a central processing unit (CPU), a semiconductor-based microprocessor, and/or other hardware devices suitable for retrieval and execution of instructions stored in machine-readable storage medium 204. Processor 202 may fetch, decode, and execute instructions 206, 208, 210, 212, and 214 to control a process of providing drugs to a patient. As an alternative or in addition to retrieving and executing instructions, processor 202 may include at least one electronic circuit that includes electronic components for performing the functionality of instructions 206, 208, 210, 212, 214, or a combination thereof.

Machine-readable storage medium 204 may be any electronic, magnetic, optical, or other physical storage device that contains or stores executable instructions. Thus, machine-readable storage medium 204 may be, for example, Random Access Memory (RAM), an EPROM, an Electrically Erasable Programmable Read-Only Memory (EEPROM), a storage device, an optical disc, and the like. In some examples, machine-readable storage medium 204 may be a non-transitory storage medium, where the term "non-transitory" does not encompass transitory propagating signals. As described in detail below, machine-readable storage medium 204 may be encoded with a series of processor executable instructions 206, 208, 210, 212, and 214 for receiving rules relating to administration of a drug to a patient, the rules specifying a timing and an amount of the drug to be administered and an identity of the patient, the rules being received from a server in communication with computing device 200 and accessible to a doctor capable of prescribing the drug and a pharmacist capable of providing the drug; producing a notification based on the timing specified in the rules; determining an identity of a person attempting to access the drug; and providing the amount of the drug based on the rules if the identity of the person is the identity of the patient.

Server communication instructions 206 may manage and control communication between computing device 200 and a server that is also in communication with and accessible to one or more doctors and one or more pharmacists. Server communication instructions 206 may receive (e.g., from a doctor and/or pharmacist via the server) rules and/or modifications to the rules relating to administration of a drug to a patient, where the rules specify information such as a timing and an amount of the drug to be administered and the identity of the patient. Server communication instructions 206 may also transmit any suitable information from computing device 200 to the server, such as information relating to the administration of the drug to the patient.

Notification instructions 208 may manage and control the production of a notification based on the timing specified in the received rules. The notification may alert the patient when it is time for the patient to take their medication. The notification may be provided in any suitable form, such as an audible notification (e.g., via a speaker associated with computing device 200), a vibration of computing device 200, a visual notification on a display associated with computing device 200, an email, a text message, a phone call, a notification on an associated wearable device, and the like.

Patient identification instructions 210 may manage and control the determination of an identity of a person attempting to access the drug associated with computing device 200. For example, patient identification instructions 210 may determine an identity of a person attempting to access the drug using a biometric scanner, such as a fingerprint scanner. Patient identification instructions 210 may receive a fingerprint scan and determine whether the received fingerprint scan matches a fingerprint scan associated with the patient to whom the drug in computing device 200 is to be administered.

Drug administration instructions 212 may manage and control the administration of a drug based on the received rules. For example, if patient identification instructions 210 determine that the person attempting to access the drug is the correct patient, drug administration instructions 212 may provide and/or dispense the proper amount of the drug to the patient in the proper manner if the drug is to be administered at that time according to the rules.

Display instructions 214 may manage and control the display of information associated with the administration of the drug. For example, display instructions 214 may cause a display associated with computing device 200 to display rules relating to the administration of the drug, a notification notifying the patient to take the drug, and the like.

Examples provided herein (e.g., methods) may be implemented in hardware, software, or a combination of both. Example systems may include a controller/processor and memory resources for executing instructions stored in a tangible non-transitory medium (e.g., volatile memory, non-volatile memory, and/or machine-readable media). Non-transitory machine-readable media can be tangible and have machine-readable instructions stored thereon that are executable by a processor to implement examples according to the present disclosure.

An example system can include and/or receive a tangible non-transitory machine-readable medium storing a set of machine-readable instructions (e.g., software). As used herein, the controller/processor can include one or a plurality of processors such as in a parallel processing system. The memory can include memory addressable by the processor for execution of machine-readable instructions. The machine-readable medium can include volatile and/or non-volatile memory such as a random access memory ("RAM"), magnetic memory such as a hard disk, floppy disk, and/or tape memory, a solid state drive ("SSD"), flash memory, phase change memory, and the like.

## Claims

1. A dispenser (100) comprising:
a processor (102) in communication with a server accessible to a doctor capable of prescribing a drug to a patient and a pharmacist capable of providing the drug to the patient, the processor (102) configured to receive instructions relating to administration of the drug to the patient, the instructions specifying a timing and an amount of the drug to be administered and an identity of the patient;
a timer (106) in communication with the processor (102), the timer (106) configured to provide a notification based on the timing specified in the instructions; and
an identifier mechanism (108) in communication with the processor (102), the identifier mechanism (108) configured to determine an identity of a person attempting to access the drug, wherein the at least one processor (102) is further configured to provide the amount of the drug based on the instructions if the identity of the person is the identity of the patient, **characterized in that**:
the processor (102) is in communication with a wearable device capable of performing bio-sensor monitoring and is configured to monitor the patient's biometrics after providing the drug to determine whether a patient has taken the drug.

2. The dispenser (100) of claim 1, wherein the instructions are remotely modifiable by the doctor or the pharmacist via the server.

3. The dispenser (100) of claim 1, wherein the instructions are based on preferences specified by the patient via the server.

4. The dispenser (100) of claim 1, wherein the processor (102) is further configured to transmit, to the server, information associated with the administration of the drug.

5. The dispenser (100) of claim 1, wherein the drug is in pill form, liquid form, or injection form.

6. The dispenser (100) of claim 1, wherein the processor (102) is further configured to receive an identifier associated with the identity of the patient and provide the drug based on the identifier.

7. The dispenser (100) of claim 1, further comprising:
a display (110) in communication with the processor (102), the display (110) capable of displaying information relating to the instructions.

8. The dispenser (100) of claim 1, wherein the processor (102) is further configured to provide an alert via email, text, phone, a speaker (112) associated with the dispenser (100), a display (110) associated with the dispenser (100), or a wearable device in communication with the processor (102), the alert being based on the timing specified in the instructions.

9. The dispenser (100) of claim 1, wherein the instructions include an instruction to modify the administration of the drug based on a missed dose.

10. A non-transitory machine-readable storage medium storing instructions that, if executed by at least one processor (102) of a computing device, cause the computing device to:
receive rules relating to administration of a drug to a patient, the rules specifying a timing and an amount of the drug to be administered and an identity of the patient, the rules being received from a server in communication with the computing device and accessible to a doctor capable of prescribing the drug and a pharmacist capable of providing the drug;
produce a notification based on the timing specified in the rules;
determine an identity of a person attempting to access the drug;
dispense the amount of the drug based on the rules if the identity of the person is the identity of the patient;
communicate with a wearable device capable of performing bio-sensor monitoring; and
monitor the patient's biometrics, by the wearable device, after providing the drug to determine whether a patient has taken the drug.

11. The non-transitory machine-readable storage medium of claim 10, wherein the rules are remotely modifiable by the doctor or the pharmacist via the server.

12. The non-transitory machine-readable storage medium of claim 10, wherein the rules are based on preferences specified by the patient via the server.

13. The non-transitory machine-readable storage medium of claim 10, wherein the instructions further cause the computing device to transmit, to the server, information associated with the administration of the drug.

14. The non-transitory machine-readable storage medium of claim 10, wherein the instructions further cause the computing device to display information relating to the rules.

15. The non-transitory machine-readable storage medium of claim 10, wherein the instructions further cause the computing device to provide an alert via email, text, phone, a speaker associated with the computing device, a display associated with the computing device, or a wearable device in communication with the computing device, the alert being based on the timing specified in the rules.

## Patentansprüche

1. Spender (100), umfassend:
einen Prozessor (102) in Verbindung mit einem Server, auf den durch einen Arzt, der dazu in der Lage ist, ein Arzneimittel an einen Patienten zu verschreiben, und durch einen Apotheker zugegriffen werden kann, der dazu in der Lage ist, dem Patienten das Arzneimittel bereitzustellen, wobei der Prozessor (102) dazu konfiguriert ist, Anweisungen zu empfangen, die sich auf die Verabreichung des Arzneimittels an den Patienten beziehen, wobei die Anweisungen einen Zeitpunkt und eine Menge des Arzneimittels, das verabreicht werden soll, und eine Identität des Patienten spezifizieren,
einen Zeitmesser (106) in Verbindung mit dem Prozessor (102), wobei der Zeitmesser (106) dazu konfiguriert ist, eine Benachrichtigung auf Grundlage des Zeitpunkts, der in den Anweisungen spezifiziert ist, bereitzustellen, und
einen Kennungsmechanismus (108) in Verbindung mit dem Prozessor (102), wobei der Kennungsmechanismus (108) dazu konfiguriert ist, eine Identität einer Person zu bestimmen, die versucht, auf das Arzneimittel zuzugreifen, wobei der wenigstens eine Prozessor (102) ferner dazu konfiguriert ist, die Menge des Arzneimittels auf Grundlage der Anweisungen bereitzustellen, wenn die Identität der Person die Identität des Patienten ist, **dadurch gekennzeichnet, dass**:
der Prozessor (102) in Verbindung mit einer tragbaren Vorrichtung steht, die in der Lage ist, Biosensor-Überwachen durchzuführen, und dazu konfiguriert ist, die Biometrik des Patienten zu überwachen, nachdem das Arzneimittel bereitgestellt worden ist, um zu bestimmen, ob ein Patient das Arzneimittel eingenommen hat.

2. Spender (100) nach Anspruch 1, wobei die Anweisungen durch den Arzt oder den Apotheker über den Server entfernt modifizierbar sind.

3. Spender (100) nach Anspruch 1, wobei die Anweisungen auf Vorlieben basieren, die durch den Patienten über den Server spezifiziert werden.

4. Spender (100) nach Anspruch 1, wobei der Prozessor (102) ferner dazu konfiguriert ist, Informationen, die der Verabreichung des Arzneimittels zugeordnet sind, an den Server zu übertragen.

5. Spender (100) nach Anspruch 1, wobei das Arzneimittel in Tablettenform, in flüssiger Form oder Injektionsform vorliegt.

6. Spender (100) nach Anspruch 1, wobei der Prozessor (102) ferner dazu konfiguriert ist, eine Kennung, die der Identität des Patienten zugeordnet ist, zu empfangen und das Arzneimittel auf Grundlage der Kennung bereitzustellen.

7. Spender (100) nach Anspruch 1, ferner umfassend:
eine Anzeige (110) in Verbindung mit dem Prozessor (102), wobei die Anzeige (110) dazu in der Lage ist, Informationen bezüglich der Anweisungen anzuzeigen.

8. Spender (100) nach Anspruch 1, wobei der Prozessor (102) ferner dazu konfiguriert ist, eine Warnung über E-Mail, SMS, Telefon, einen Lautsprecher (112), der dem Spender (100) zugeordnet ist, eine Anzeige (110), die dem Spender (100) zugeordnet ist, oder eine tragbare Vorrichtung in Verbindung mit dem Prozessor (102) bereitzustellen, wobei die Warnung auf dem in den Anweisungen spezifizierten Zeitpunkt basiert.

9. Spender (100) nach Anspruch 1, wobei die Anweisungen eine Anweisung zum Modifizieren der Verabreichung des Arzneimittels auf Grundlage einer verpassten Dosis beinhalten.

10. Nichtflüchtiges maschinenlesbares Speichermedium, das Anweisungen speichert, die, wenn sie durch den wenigstens einen Prozessor (102) einer Rechenvorrichtung ausgeführt werden, die Rechenvorrichtung veranlassen zum:
Empfangen von Regeln bezüglich der Verabreichung eines Arzneimittels an einen Patienten, wobei die Regeln einen Zeitpunkt und eine Menge des Arzneimittels, das verabreicht werden soll, und eine Identität des Patienten spezifizieren, wobei die Regeln von einem Server in Verbindung mit der Rechenvorrichtung empfangen werden und auf die durch einen Arzt, der in der Lage ist, das Arzneimittel zu verschreiben, und durch einen Apotheker zugegriffen werden kann, der in der Lage ist, das Arzneimittel bereitzustellen,
Produzieren einer Benachrichtigung auf Grundlage des in den Regeln spezifizierten Zeitpunkts,
Bestimmen einer Identität einer Person, die versucht, auf das Arzneimittel zuzugreifen;
Abgeben der Menge des Arzneimittels auf Grundlage der Regel, wenn die Identität der Person die Identität des Patienten ist,
Verbinden mit einer tragbaren Vorrichtung, die in der Lage ist, ein Biosensor-Überwachen durchzuführen, und
Überwachen der Biometrik des Patienten durch die tragbare Vorrichtung, nachdem das Arzneimittel bereitgestellt worden ist, um zu bestimmen, ob ein Patient das Arzneimittel eingenommen hat.

11. Nichtflüchtiges maschinenlesbares Speichermedium nach Anspruch 10, wobei die Regeln durch den Arzt oder den Apotheker über den Server entfernt modifizierbar sind.

12. Nichtflüchtiges maschinenlesbares Speichermedium nach Anspruch 10, wobei die Regeln auf den Vorlieben, die durch den Patienten über den Server spezifiziert werden, basieren.

13. Nichtflüchtiges maschinenlesbares Speichermedium nach Anspruch 10, wobei die Anweisungen ferner die Rechenvorrichtung veranlassen, Informationen, die der Verabreichung des Arzneimittels zugeordnet sind, an den Server zu übertragen.

14. Nichtflüchtiges maschinenlesbares Speichermedium nach Anspruch 10, wobei die Anweisungen ferner die Rechenvorrichtung veranlassen, Informationen anzuzeigen, die sich auf die Regeln beziehen.

15. Nichtflüchtiges maschinenlesbares Speichermedium nach Anspruch 10, wobei die Anweisungen ferner die Rechenvorrichtung veranlassen, eine Warnung über E-Mail, SMS, Telefon, einen Lautsprecher, der der Rechenvorrichtung zugeordnet ist, eine Anzeige, die der Rechenvorrichtung zugeordnet ist, oder eine tragbare Vorrichtung in Verbindung mit der Rechenvorrichtung bereitzustellen, wobei die Warnung auf dem in den Regeln spezifizierten Zeitpunkt basiert.

## Revendications

1. Distributeur (100) comportant :
un processeur (102) en communication avec un serveur accessible par un docteur en mesure de prescrire un médicament à un patient et par un pharmacien en mesure de fournir le médicament au patient, le processeur (102) étant configuré pour recevoir des instructions se rapportant à l'administration du médicament au patient, les instructions spécifiant un moment précis et une quantité du médicament devant être administré et une identité du patient ;
une minuterie (106) en communication avec le processeur (102), la minuterie (106) étant configurée pour fournir une notification sur la base du moment précis spécifié dans les instructions ; et
un mécanisme d'identification (108) en communication avec le processeur (102), le mécanisme d'identification (108) étant configuré pour déterminer une identité d'une personne qui tente d'avoir accès au médicament, dans lequel ledit au moins un processeur (102) est par ailleurs configuré pour fournir la quantité du médicament sur la base des instructions si l'identité de la personne est l'identité du patient, **caractérisé en ce que** :
le processeur (102) est en communication avec un objet personnel connecté en mesure d'effectuer une surveillance par biocapteur et est configuré pour surveiller la biométrie du patient après avoir fourni le médicament pour déterminer si un patient a pris le médicament.

2. Distributeur (100) selon la revendication 1, dans lequel les instructions sont modifiables à distance par le docteur ou par le pharmacien par le biais du serveur.

3. Distributeur (100) selon la revendication 1, dans lequel les instructions sont basées sur des préférences spécifiées par le patient par le biais du serveur.

4. Distributeur (100) selon la revendication 1, dans lequel le processeur (102) est par ailleurs configuré pour transmettre, au serveur, des informations associées à l'administration du médicament.

5. Distributeur (100) selon la revendication 1, dans lequel le médicament se présente sous la forme de pilule, la forme de liquide, ou la forme d'injection.

6. Distributeur (100) selon la revendication 1, dans lequel le processeur (102) est par ailleurs configuré pour recevoir un identifiant associé à l'identité du patient et pour fournir le médicament sur la base de l'identifiant.

7. Distributeur (100) selon la revendication 1, comportant par ailleurs :
un écran d'affichage (110) en communication avec le processeur (102), l'écran d'affichage (110) étant en mesure d'afficher des informations se rapportant aux instructions.

8. Distributeur (100) selon la revendication 1, dans lequel le processeur (102) est par ailleurs configuré pour fournir une alerte par le biais d'un courriel, d'un texte, d'un téléphone, d'un haut-parleur (112) associé au distributeur (100), d'un écran d'affichage (110) associé au distributeur (100), ou d'un objet personnel connecté en communication avec le processeur (102), l'alerte se faisant sur la base du moment précis spécifié dans les instructions.

9. Distributeur (100) selon la revendication 1, dans lequel les instructions comprennent une instruction pour modifier l'administration du médicament sur la base d'une dose manquée.

10. Support de stockage non transitoire lisible par machine stockant des instructions qui, si elles sont exécutées par au moins un processeur (102) d'un dispositif informatique, amènent le dispositif informatique à :
recevoir des règles se rapportant à l'administration d'un médicament à un patient, les règles spécifiant un moment précis et une quantité du médicament devant être administré et une identité du patient, les règles étant reçues en provenance d'un serveur en communication avec le dispositif informatique et accessibles par un docteur en mesure de prescrire le médicament et par un pharmacien en mesure de fournir le médicament ;
produire une notification sur la base du moment précis spécifié dans les règles ;
déterminer une identité d'une personne qui tente d'avoir accès au médicament ;
distribuer la quantité du médicament sur la base des règles si l'identité de la personne est l'identité du patient ;
communiquer avec un objet personnel connecté en mesure d'effectuer une surveillance par biocapteur ; et
surveiller la biométrie du patient, par l'objet personnel connecté, après avoir fourni le médicament pour déterminer si un patient a pris le médicament.

11. Support de stockage non transitoire lisible par machine selon la revendication 10, dans lequel les règles sont modifiables à distance par le docteur ou par le pharmacien par le biais du serveur.

12. Support de stockage non transitoire lisible par machine selon la revendication 10, dans lequel les règles sont basées sur des préférences spécifiées par le patient par le biais du serveur.

13. Support de stockage non transitoire lisible par machine selon la revendication 10, dans lequel les instructions amènent par ailleurs le dispositif informatique à transmettre, au serveur, des informations associées à l'administration du médicament.

14. Support de stockage non transitoire lisible par machine selon la revendication 10, dans lequel les instructions amènent par ailleurs le dispositif informatique à afficher des informations se rapportant aux règles.

15. Support de stockage non transitoire lisible par machine selon la revendication 10, dans lequel les instructions amènent par ailleurs le dispositif informatique à fournir une alerte par le biais d'un courriel, d'un texte, d'un téléphone, d'un haut-parleur associé au dispositif informatique, d'un écran d'affichage associé au dispositif informatique, ou d'un objet personnel connecté en communication avec le dispositif informatique, l'alerte se faisant sur la base du moment précis spécifié dans les règles.
